# EUROPEAN PATENT SPECIFICATION

(11) **EP 1 850 750 B1**
(45) Date of publication and mention of the grant of the patent: **21.07.2010**
(21) Application number: 06701627.9
(22) Date of filing: 30.01.2006
(51) Int. Cl.: A61B 5/15

(54) **AN AUTOMATIC BLOOD SAMPLING DEVICE**
AUTOMATISCHE BLUTPROBENAHMEVORRICHTUNG
DISPOSITIF DE PRELEVEMENT SANGUIN AUTOMATIQUE

(30) Priority: 31.01.2005 US 647778 P
(43) Date of publication of application: 07.11.2007
(73) Proprietor: Aarhus Universitet, 8000 Aarhus C (DK)
(72) Inventor: FONSS, Ander, DK-9000 Aalborg (DK)
(74) Representative: Inspicos A/S
(86) International application number: PCT/DK2006/000047
(87) International publication number: WO 2006/079345

(56) References cited:
- US-A- 4 077 395
- US-A- 5 325 867
- US-A1- 2002 025 255

## Description

### FIELD OF THE INVENTION

The present invention relates to an automatic blood sampling device adapted to take blood samples of animals without stressing the animals. The present invention further relates to an automatic blood sampling device where the risk of blood coagulation within the device, causing the device to malfunction, is significantly reduced.

### BACKGROUND OF THE INVENTION

Various types of automatic blood sampling devices have been proposed over the recent years.

One example of such prior art blood sampling device has been suggested by P. J. Goddard et al. in their paper "Automatic blood sampling equipment for use in studies of animal physiology" published in Animal Science 1998, 66, page 769-775. In this paper Goddard et al. suggest an automatic blood sampling device using a 16 position valve. Blood samples are pumped from a vein catheter part and to a plurality of collection tubes. By controlling the 16 position valve a blood sample can be directed to an empty collection tube.

It is a disadvantage of the device suggested by Goddard et al. that due to the presence of the 16 positions valve the proposed device is difficult to keep free from coagulated blood which, over a relatively short time period, will cause malfunctions of the device. Such malfunctions could be that a preprogrammed series of samples to be taken is not completed.

The first part of present claim 1 is known from US-A-5325867.

It is an object of the present invention to provide an automatic blood sampling device where the risk of blood coagulation within the device is minimized.

It is a further object of the present invention to provide an automatic blood sampling device having a significantly simplified mechanical construction.

### SUMMARY OF THE INVENTION

The above-mentioned objects are complied with by providing, in a first aspect, an automatic blood sampling device as defined in claim 1.

The penetration member may be adapted to penetrate a flexible member, such as a rubber membrane, of the storage container which may be a vacuum tube. By essentially unbroken is meant that any liquid is free to flow between the catheter part and the penetration member without meeting any obstacles. Thus, the liquid needs not to pass arrangements such as flow splitters and/or other valve-like arrangements.

The penetration member may in principle comprise any kind of needle or canulla capable of transporting blood. The catheter part and needle/canulla may be interconnected by a single tube section, or by a tube section being constituted by one or more tube sections. Such one or more tube sections may have different dimensions such as diameters and/or lengths. Even further, the one or more tube sections may have different optical properties - for example, one section may be optically transparent, whereas another section may be non-transparent.

The catheter part may be constituted by a tube section having a length between 5 and 150 cm. The length of the catheter part varies with the size of the animal in which the catheter is to be positioned. Generally speaking, the smaller the animal, the shorter the catheter part. The catheter part is adapted to be positioned within a vein of the animal to deliver the blood sample. In case of the big animal, such as a cow, the device according to the present invention will be carried by the animal, for example by positioning the device in a sack. In case of the small animal, such as a mouse, the device according to the present invention will be stationary mounted with a tube section between the mouse and the device.

The needle/canulla may be movably mounted in relation to the storage container. Such movable arrangement of the needle/canulla may be provided by arranging the needle/canulla on a carriage or wagon, the carriage or wagon being adapted to perform at least a translational movement in relation to the storage container. The translational movement of the carriage or wagon may be along a centre line defined by the storage container. The needle/canulla may be aligned with said centre line when the needle/canulla penetrates the flexible member of the storage container in order to deliver a blood sample to the storage container. The storage container may contain a blood sample with a volume of between 0.5 and 6 ml, such as approximately 4.5 ml.

The automatic blood sampling device may further comprise a first and a second sensor element, wherein the first sensor element may be adapted to provide a first position signal when the carriage is at its withdrawn position where the canulla is separated from the storage container, and wherein the second sensor element may be adapted to provide a second position signal when the carriage is at its front position where the canulla has penetrated the flexible member of the storage container. The first and second sensor elements may be electrical switches, i.e. a switch that is short-circuited when the carriage is in contact with the switch. Alternatively, the first and second sensor elements may be optically based sensors where an optical beam is influenced, such as broken or allowed to pass, when the carriage is at predetermined positions, such as at its end positions.

The carriage or wagon may be driven or moved between its end positions by electrical or hydraulic means. In case of electrical means, an electric motor, such as a controlled DC-motor, a servo or a stepper motor, may be used to move the carriage or wagon. In case of hydraulic means, a piston may move the carriage or wagon between its end positions.

The automatic blood sampling device may further comprise additional storage containers so that the device comprises a plurality of storage containers. These storage containers may be arranged in a rotatably mounted drum. The rotatably mounted drum may comprise a first and a second substantially circular disc, wherein the first and second discs are mutually aligned and mounted on a rotatable axle operatively connected to an electrical motor, such as a controlled DC-motor or stepper motor. Each of the substantially circular discs may comprise a number of arrangements adapted to support the plurality of storage containers. These arrangements may comprise through-going openings in one or both discs. In this way storage containers may be mounted in the drum by positioning each of the one or more storage containers in corresponding pairs of openings so that one end of a storage container rests in an opening formed in the first disc, whereas the other end of the storage container rests in an opening formed in the second disc.

The automatic blood sampling device may further comprise an alignment mechanism adapted to axially align the penetration member with a storage container. The alignment mechanism may comprise means for generating an align signal when the storage container is in axial alignment with the penetration member. When axial alignment of the storage container and the penetration member is established, the penetration member may safely enter the storage container by penetrating the flexible member sealing the storage container. In order to penetrate the flexible member a pressure corresponding to approximately 1 kilogram needs to be applied to the penetration member.

The alignment mechanism may comprise an optical emitter and an optical receiver, the optical emitter being arranged so as to at least partly couple a light beam through transparent portions of a disc. The receiver may be arranged so as to receive and detect transmitted parts of the light beam. Such transparent portions are provided in alignment with through-going openings in one of the discs. Thus, the transparent portions may be radially aligned with the through-going openings adapted to support for example the bottom end of a storage container. The optical emitter may be a LED, a laser or any other light source. The wavelength may be in the visible range or in the near infra-red range. The receiver may be any kind of photosensitive device, such as a photosensitive diode.

The alignment mechanism may also be implemented by other means. For example, the position of the drum may be determined using magnetic means where discrete magnets positioned on a disc actuate a switch fixedly positioned relative to the drum. Other mechanical means, such as mechanical systems relying on physical contact between a rotating part of the drum and a fixedly positioned sensor/switch, may also be implemented.

The align signal may be generated when the optical receiver receives an optical signal from the optical emitter, the optical signal being transmitted from the optical emitter and to the optical receiver through a through-going opening in a third substantially circular disc.

The rotatably mounted drum may be adapted to accommodate 14 storage containers, a waste container, and a reservoir adapted to contain an anticoagulant, such as heparin. Obviously, the drum may be adapted to accommodate a different number of storage containers which may be smaller or larger than 14. An alignment mechanism may be provided for each of the storage containers, the waste container and the reservoir.

The pump means may comprise a peristaltic pump positioned between the catheter part and the penetration member. The automatic blood sampling device according to the present invention may further comprise a central control unit for controlling the operation of the blood sampling device. By operation is meant that the control unit controls the operation of the pump means, the movement of the penetration member, the rotation of the drum holding the storage containers etc. As control parameters, the control unit applies and processes the position signals from the first and second sensor elements and the align signal. It may also be that the automatic blood sampling device may be controlled in a feed-forward manner which implies that a smaller number of control signals, and thereby sensor elements, are required. Thus, the carriage, the drum and the pump may be controlled by applying a feed-forward regulation approach.

The automatic blood sampling device according to the present invention may further comprise electronic storage means adapted to store readable programs, said readable programs determining, when executed by the central control unit, the operation of the blood sampling device. Thus, the control unit may be preprogrammed to perform a set of the measurements in that the control unit may configure the device to take number of blood samples with a predetermined time interval. Also, the volume of the each of the blood samples to be taken may be preprogrammed into the device.

### BRIEF DESCRIPTION OF THE INVENTION

The invention will now by described in further details with reference to the accompanying figures, wherein
- Fig. 1: shows the overall concept of the present invention,
- Fig. 2: shows the moveable carriage/slide of the present invention in a first embodiment,
- Fig. 3: shows the moveable carriage/slide of the present invention in a second embodiment, and
- Fig. 4: shows a set of polycarbonate discs for holding a plurality of vacuum types.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within scope of the invention as defined by the appended claims.

### DETAILED DESCRIPTION OF THE INVENTION

Generally speaking the present invention relates to an automatic blood sampling device having a very simple mechanical construction. The main advantage of the present invention lies in that there is an essentially unbroken path from a catheter part (inserted in a vein of an animal or human) to a canulla. The canulla is adapted to penetrate a rubber membrane of a vacuum tube so that a blood sample can be pumped into the vacuum tube. The pump provided in the present invention is as peristaltic pump which leaves the path from the catheter part to the canulla unbroken. The main advantage of having an essentially unbroken path between the catheter part and the canulla is that an anti coagulant, such as heparin, may easily be flushed through all parts of the blood sampling device. Thus, due to its simple mechanical construction, the blood sampling device according to the present invention may easily be cleaned whereby the risk of malfunctioning due to coagulated blood in the device is significantly reduced.

The blood sampling device according to the present invention may be used as a portable device being carried by a big animal or a human, or it may be used as a stationary device where only the catheter part and part of an intermediate tube section is being carried by an animal, for example a small animal like a mouse or a rat.

The blood sampling device according to the present invention is a fully automatic device which can be preprogrammed to take a series of blood samples. For example, the device according to the present invention may be preprogrammed to take a certain number of blood samples within a given time of period. This allows blood samples to be taken without stressing the animals whereby more correctly and trustily measurements can be taken. Also, the blood sampling device according to the present invention can be controlled using wireless communication between a portable or stationary control unit and a receiver and/or transmitter unit operatively connected with the blood sampling device. Thus, data or control commands may be wirelessly transmitted to and/or from the blood sampling device.

In its most general aspect, the present invention relates to a blood sampling device 1 having a catheter part 2 adapted to be inserted in a vein of an animal or human, an injection needle/canulla 3 mounted on a moveable carriage 4, an intermediate tube section 5 interconnecting the catheter part and the needle/canulla 3 and a peristaltic pump 6 for pumping the blood from the catheter part and into one of a plurality of vacuum types 7.

In case blood samples are to be taken on a cow or another animal of approximately the same size the catheter part 2 has a length of 15-20 cm, whereas the tube section 5 has a length of around 160 cm. The inner diameter of the catheter part 2 is 1.016 mm. The needle/canulla 3 is an injection needle of the type 21G capable of penetrating a sealing arrangement, such as a rubber membrane, of the vacuum types. It should be noted that the lengths of the catheter part 2 and the intermediate tube section 5 may easily be changed if blood samples are to be taken on a smaller animal.

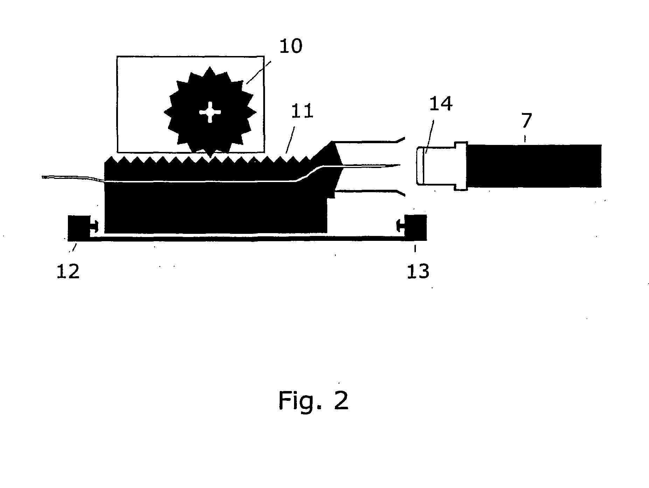

The needle/canulla 3 is mounted on the carriage 4 which is capable performing translational movements over distances between 15 mm and 25 mm. The carriage is driven by a controlled DC-motor having a gear wheel mounted on its motor axle. The teeth 10 of the gear wheel mesh with a plurality of grooves or indentations 11 formed in a surface of the carriage - see Fig. 2 - whereby a rotational movement of the motor shaft and gear wheel is converted to a translational movement of the carriage. A pressure switch 12 and 13 is provided at each end of the traveling path of the carriage. Pressure switch 12 provides a backward stop signal when the carriage reaches its outermost back position, whereas pressure switch 13 provides a forward stop signal when the carriage reaches its outermost front position. The backward and forward stop signals are provided to a central control unit for further processing. When the carriage has reached its outermost front position, the needle/canulla has fully penetrated the rubber membrane 14 of the vacuum tube 7 whereby a blood sample can be deposited inside the vacuum tube 7. Similarly, when the position of the carriage generates a backward stop signal, the central control unit receives a backward stop signal which means that the needle/canulla is fully retracted, and thereby fully disengaged from the vacuum tube.

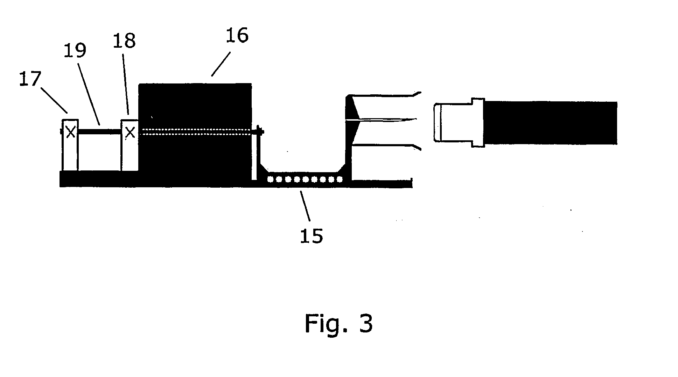

In Fig. 3 an alternative embodiment is depicted. As seen the carriage 15 is moved by a stepper motor 16 and the position of the carriage 15 relative to the motor is determined by the use of two optically based sensors 17, 18 positioned on the opposite side of the stepper motor 16 (relative to the carriage). A non-rotating threaded rod 19 is moved in and out of the motor 16 in response to signals applied to the motor. One end of the threaded rod 19 is attached to the carriage 15 whereby a translational movement of the threaded rod 19 causes the carriage 15 to perform a corresponding translational movement. The end positions of the carriage are detected by the two optically based sensors 17, 18 in that the threaded rod 19 influences an optical beam of each sensor.

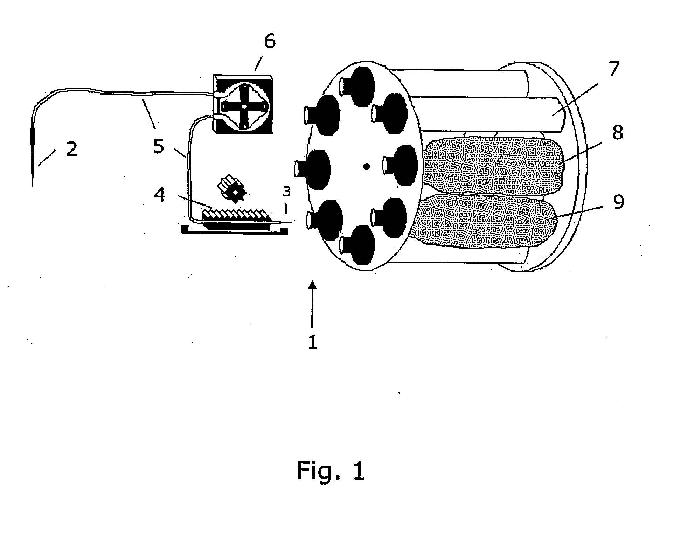

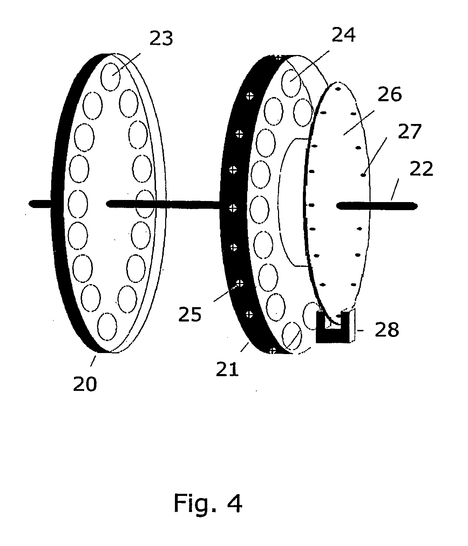

Returning to Fig. 1 the blood sampling device has a rotatably mounted drum which is capable of housing a plurality of vacuum tubes and at least one waste container 8 and at least one container 9 for containing an anti coagulant, such as heparin. The rotatably mounted drum is described in further details in connection with Fig. 4.

The blood sampling device according to the present invention is operated in the following manner:
Before a blood sample is taken the catheter 2, the tube section 5 and the needle/canulla 3 is filled with an anti coagulant, such as heparin. When a blood sample is to be taken, the anti coagulant in the catheter, the tube section and the needle/canulla is pumped into the waste container 8 and the catheter, the tube section and needle/canulla is then filled with blood. A small amount of blood is pumped into the waste container to ensure that the catheter, the tube section and needle/canulla is completely filled with blood, and only blood. When this has been achieved, the needle/canulla is withdrawn from the waste container and the drum is rotated until an empty vacuum tube is aligned with the blood filled needle/canulla. The carriage is then moved forward and towards the empty vacuum tube so that the needle/canulla penetrates the rubber membrane of the empty vacuum tube. When the carriage reaches its front stop a front stop signal is generated and a blood sample is pumped into the empty vacuum tube. Typically, blood samples have volumes of between 0.5 and 6 milliliters. After the blood sample has been pumped into the vacuum tube the carriage is moved backwards until a backward stop signal is generated. In this position of the carriage the needle/canulla is separated from the rubber membrane. The catheter, the tube section and the needle/canulla is however stilled filled with fresh blood. In order to avoid coagulation of the blood inside the before-mentioned parts of the sampling device the drum is rotated and the needle/canulla is brought into alignment with the container containing the anti coagulant. The carriage pushes the needle/canulla through the rubber membrane of the container containing the anti coagulant where after the needle/canulla, the tube section and the catheter are flushed with the anti coagulant causing the blood in the catheter part, the tube section and the needle/canulla to be pumped back into the animal or human. With the catheter, the tube section and the needle/canulla filled with the anti coagulant the blood sampling device is ready to take a new blood sample. The process of taking a blood sample and flushing the system with an anti coagulant, such as heparin, typical takes about 25 seconds.

The central control unit controls the overall performance and operation of the blood sampling device. Thus, the control unit processes the various signals from sensors installed in the blood sampling device, such as the stop signals from the pressure sensors at the carriage and an align signal for aligning the needle/canulla with the vacuum tubes, waste container or the anti coagulant container positioned in the drum. Furthermore, the control unit may be preprogrammed to take a series of blood samples. For example, the control unit can be preprogrammed to take a number of samples within a certain time period - for example, blood samples may be taken with a one hour interval until a total of 10 blood samples have been taken. The number of samples to be taken and the time interval between the samples may in principle be chosen arbitrary. Thus, the blood sampling device according to the present invention is well suited to be mounted in a sack and positioned on the back of an animal, such as a cow or a horse, or a human. Blood samples may also be taken from smaller animals if the blood sampling device is positioned on for example a table. In this stationary setup, only the catheter part and part of the tube section is carried by the animal.

Fig. 4 shows the drum of Fig. 1 in further details. As seen the drum comprises a pair of discs 20 and 21 fabricated in a polymer-based material. Both discs are rotatably mounted on a common axle 22 which as operatively connected to the motor shaft of a DC motor. The connection between the axle 22 and the motor axle may be a direct connection or they may be connected via a gear arrangement. The drum rotates with a speed of 6-8 turns per minute. The front disc 20 has a thickness of 6 mm whereas the back disc 21 has a thickness of 9 mm. The vacuum tubes to be loaded into the drum are loaded from the front disc side.

The two discs 20 and 21 comprise a number of mounting holes 23 and 24 each being adapted to hold a portion of a vacuum tube. The discs 20 and 21 are arranged so that they form pairs of aligned mounting holes. This means that a vacuum tube inserted in the drum is supported by two mounting openings - an opening in the back disc 21 supports the back part of the vacuum tube whereas an opening in the front disc 20 supports the front part of the same vacuum tube. A vacuum tube inserted in this way is kept in position by a small screw 25 inserted in a threaded opening or hole formed in the back disc 21. Alternatively, the vacuum tube could be fixated by a similar arrangement formed in the front disc 21.

To keep track of the positions of a plurality of vacuum tubes mounted in the drum a non-transparent disc 26 having a plurality of discrete transparent areas 27 is mounted on the same axle 22 as the discs 20 and 21. Obviously, the discrete transparent areas 27, which typically are implemented as through-going openings, must be aligned with the mounting openings. A sensor unit 28 comprising an optical emitter and an optical receiver generates an align signal when an optical signal is transmitted through the disc 26. This align signal is provided to the central control unit for further processing.

Additional sensors, such as a sensor for determining whether an at least partly transparent liquid is in the tube section may also be provided in the device. Using such additional sensor it may be controlled whether blood or heparin, or a combination thereof, is in the tube section at the position of the additional sensor. Such additional sensor could be an optical-based sensor where a light beam is directed through a transparent tube section.

In addition a sensor for measuring temperature may be provided. The temperature sensor may measure the air temperature in the device and/or it may measure the actual temperature of the sample. The temperature sensor may be combined with means for establishing a temperature log whereby the air and/or blood temperature may be monitored over time.

## Claims

1. An automatic blood sampling device comprising
- a catheter part (1) being operatively connected to a penetration member (3), the operatively connection between the catheter part and the penetration member being provided via one or more tube sections (5) forming an essentially unbroken path between the catheter part and the penetration member, and
- pump means (6) being adapted to pump blood through the catheter part and the penetration member and into a storage container (7), the storage container being adapted to contain a blood sample, **characterized in that** the pump means further being adapted to flush the essentially unbroken path comprising the catheter part, the one or more tube sections and the penetration member with an anti coagulant from another container (9) causing blood in the catheter part, the one or more tube sections and the penetration member to be pumped back into an animal or human.

2. An automatic blood sampling device according to claim 1, wherein the penetration member comprises a canulla adapted to penetrate a flexible member of the storage container.

3. An automatic blood sampling device according to claim 2, wherein the canulla is movably mounted in relation to the storage container.

4. An automatic blood sampling device according to claim 3, wherein the canulla is arranged on a carriage, the carriage being adapted to perform a translational movement in relation to the storage container, the translational movement of the carriage being along a centre line of the storage container, wherein the canulla is aligned with said centre line.

5. An automatic blood sampling device according to claim 4, further comprising a first and a second sensor element, wherein the first sensor element is adapted to provide a first position signal when the carriage is at its withdrawn position where the canulla is separated from the storage container, and wherein the second sensor element is adapted to provide a second position signal when the carriage is at its front position where the canulla has penetrated the flexible member of the storage container.

6. An automatic blood sampling device according to any of the preceding claims, wherein the device comprises a plurality of storage containers arranged in a rotatably mounted drum.

7. An automatic blood sampling device according to claim 6, wherein the rotatably mounted drum comprises a first and a second substantially circular disc, the discs being mutually aligned and mounted on a rotatable axle operatively connected to an electrical motor.

8. An automatic blood sampling device according to claim 7, wherein each of the substantially circular discs comprises a number of arrangements adapted to support the plurality of storage containers.

9. An automatic blood sampling device according to claim 8, wherein the arrangements for supporting the plurality of storage containers comprise through-going openings in the discs so that one storage container is fixated in the drum by penetrating a through-going opening in the first disc and penetrating an associated through-going opening in the second disc.

10. An automatic blood sampling device according to any of claims 7-9, further comprising an alignment mechanism adapted to axially align the penetration member with a storage container, the alignment mechanism comprising means for generating an align signal when the storage container is in axial alignment with the penetration member.

11. An automatic blood sampling device according to claim 10, wherein the alignment mechanism comprises an optical emitter and an optical receiver, the optical emitter and receiver being arranged so as to be able to couple a light beam through transparent portions of a disc and detect transmitted parts of said light beam.

12. An automatic blood sampling device according to claim 11, wherein transparent portions are provided in alignment with the through-going openings in one of the discs.

13. An automatic blood sampling device according to claim 12, wherein the align signal is generated when the optical receiver receives at least part of a transmitted optical signal, the optical signal being transmitted from the optical emitter and to the optical receiver through a through-going opening in a third substantially circular disc.

14. An automatic blood sampling device according to any of claims 6-13, wherein the rotatably mounted drum is adapted to accommodate 14 storage containers, a waste container, and a reservoir adapted to contain the anti coagulant, such as heparin.

15. An automatic blood sampling device according to any of the preceding claims, wherein the pump means comprises a peristaltic pump.

16. An automatic blood sampling device according to any of the preceding claims, further comprising a central control unit for controlling the operation of the blood sampling device.

17. An automatic blood sampling device according to claim 16, further comprising electronic storage means adapted to store readable programs, said readable programs determining, when executed by the central control unit, the operation of the blood sampling device.

## Patentansprüche

1. Automatische Blutentnahmevorrichtung, umfassend
- ein Katheterteil (1), das wirksam mit einem Durchdringungselement (3) verbunden ist, wobei die wirksame Verbindung zwischen dem Katheterteil und dem Durchdringungselement über einen oder mehrere Schlauchabschnitte (5) bereitgestellt wird, die einen im Wesentlichen ununterbrochenen Weg zwischen dem Katheterteil und dem Durchdringungselement bilden, und
- Pumpmittel (6), die zum Pumpen von Blut durch das Katheterteil und das Durchdringungselement und in einen Aufbewahrungsbehälter (7) eingerichtet sind, wobei der Aufbewahrungsbehälter zum Fassen einer Blutprobe eingerichtet ist, **dadurch gekennzeichnet, dass** die Pumpmittel weiterhin zum Spülen des im Wesentlichen ununterbrochenen Wegs umfassend das Katheterteil, den einen oder die mehreren Schlauchabschnitte und das Durchdringungselement mit einem Gerinnungshemmer aus einem anderen Behälter (9) eingerichtet sind, wodurch Blut in dem Katheterteil, dem einen oder den mehreren Schlauchabschnitten und dem Durchdringungselement zurück in ein Tier oder einen Menschen gepumpt wird.

2. Automatische Blutentnahmevorrichtung nach Anspruch 1, wobei das Durchdringungselement eine Kanüle umfasst, die zum Durchdringen eines flexiblen Elements des Aufbewahrungsbehälters eingerichtet ist.

3. Automatische Blutentnahmevorrichtung nach Anspruch 2, wobei die Kanüle beweglich in Bezug auf den Aufbewahrungsbehälter angebracht ist.

4. Automatische Blutentnahmevorrichtung nach Anspruch 3, wobei die Kanüle auf einem Schlitten angeordnet ist, wobei der Schlitten zur Durchführung einer Translationsbewegung in Bezug auf den Aufbewahrungsbehälter eingerichtet ist, wobei die Translationsbewegung des Schlittens entlang einer Mittellinie des Aufbewahrungsbehälters erfolgt, wobei die Kanüle an der Mittellinie ausgerichtet ist.

5. Automatische Blutentnahmevorrichtung nach Anspruch 4, weiterhin umfassend ein erstes und ein zweites Sensorelement, wobei das erste Sensorelement zur Bereitstellung eines ersten Positionssignals eingerichtet ist, wenn sich der Schlitten in seiner zurückgezogenen Position befindet, in der die Kanüle von dem Aufbewahrungsbehälter getrennt ist, und wobei das zweite Sensorelement zur Bereitstellung eines zweiten Positionssignals eingerichtet ist, wenn sich der Schlitten in seiner vorderen Position befindet, in der die Kanüle das flexible Element des Aufbewahrungsbehälters durchdrungen hat.

6. Automatische Blutentnahmevorrichtung nach einem der vorhergehenden Ansprüche, wobei die Vorrichtung eine Mehrzahl an Aufbewahrungsbehälter umfasst, die in einer drehbar angebrachten Trommel eingerichtet sind.

7. Automatische Blutentnahmevorrichtung nach Anspruch 6, wobei die drehbar angebrachte Trommel eine erste und eine zweite im Wesentlichen kreisförmige Scheibe umfasst, wobei die Scheiben aneinander ausgerichtet und an einer drehbaren Welle montiert sind, die wirksam mit einem Elektromotor verbunden ist.

8. Automatische Blutentnahmevorrichtung nach Anspruch 7, wobei jede der im Wesentlichen kreisförmigen Scheiben eine Anzahl von Einrichtungen umfasst, die zum Halten der Mehrzahl an Aufbewahrungsbehältern eingerichtet sind.

9. Automatische Blutentnahmevorrichtung nach Anspruch 8, wobei die Einrichtungen zum Halten der Mehrzahl an Aufbewahrungsbehältern Durchgangsöffnungen in den Scheiben umfassen, sodass ein Aufbewahrungsbehälter durch Durchdringen einer Durchgangsöffnung in der ersten Scheibe und Durchdringen einer entsprechenden Durchgangsöffnung in der zweiten Scheibe in der Trommel fixiert wird.

10. Automatische Blutentnahmevorrichtung nach einem der Ansprüche 7-9, weiterhin umfassend einen Ausrichtmechanismus, der zum axialen Ausrichten des Durchdringungselements an einem Aufbewahrungsbehälter eingerichtet ist, wobei der Ausrichtmechanismus Mittel zum Erzeugen eines Ausrichtsignals umfasst, wenn der Aufbewahrungsbehälter axial zum Durchdringungselement ausgerichtet ist.

11. Automatische Blutentnahmevorrichtung nach Anspruch 10, wobei der Ausrichtmechanismus einen optischen Sender und einen optischen Empfänger umfasst, wobei der optische Sender und der optische Empfänger derart angeordnet sind, dass sie einen Lichtstrahl durch durchsichtige Abschnitte einer Scheibe koppeln und übertragene Teile des Lichtstrahls erfassen.

12. Automatische Blutentnahmevorrichtung nach Anspruch 11, wobei die durchsichtigen Abschnitte an den Durchgangsöffnungen in einer der Scheiben ausgerichtet vorgesehen sind.

13. Automatische Blutentnahmevorrichtung nach Anspruch 12, wobei das Ausrichtsignal erzeugt wird, wenn der optische Empfänger mindestens einen Teil eines übertragenen optischen Signals empfängt, wobei das optische Signal von dem optischen Sender durch eine Durchgangsöffnung in einer dritten im Wesentlichen kreisförmigen Scheibe an den optischen Empfänger übertragen wird.

14. Automatische Blutentnahmevorrichtung nach einem der Ansprüche 6-13, wobei die drehbar angebrachte Trommel zur Aufnahme von 14 Aufbewahrungsbehältern, einem Abfallbehälter und einem zum Fassen des Gerinnungsmittels, wie Heparin, eingerichteten Reservoir eingerichtet ist.

15. Automatische Blutentnahmevorrichtung nach einem der vorhergehenden Ansprüche, wobei die Pumpenmittel eine peristaltische Pumpe umfassen.

16. Automatische Blutentnahmevorrichtung nach einem der vorhergehenden Ansprüche, weiterhin umfassend eine zentrale Steuereinheit zur Steuerung des Betriebs der Blutentnahmevorrichtung.

17. Automatische Blutentnahmevorrichtung nach Anspruch 16, weiterhin umfassend elektronische Speichermittel, die zum Speichern lesbarer Programme eingerichtet sind, wobei die lesbaren Programme beim Ausführen durch die zentrale Steuereinheit den Betrieb der Blutentnahmevorrichtung festlegen.

## Revendications

1. Dispositif de prélèvement de sang automatique comprenant
- une partie cathéter (1) raccordée de manière fonctionnelle à un élément de pénétration (3), le raccordement fonctionnel entre la partie cathéter et l'élément de pénétration étant assuré par un ou plusieurs tronçons de tube (5) formant une voie essentiellement continue entre la partie cathéter et l'élément de pénétration, et
- un moyen de pompage (6) approprié pour pomper le sang au travers de la partie cathéter et de l'élément de pénétration jusqu'à un récipient de stockage (7), le récipient de stockage étant approprié pour contenir un prélèvement sanguin, **caractérisé en ce que** le moyen de pompage est également approprié pour rincer la voie essentiellement continue comprenant la partie cathéter, l'un ou plusieurs tronçons de tube et l'élément de pénétration avec un anticoagulant provenant d'un autre récipient (9) en refoulant ainsi le sang présent dans la partie cathéter, l'un ou plusieurs tronçons de tube et l'élément de pénétration dans un animal ou un humain.

2. Dispositif de prélèvement de sang automatique selon la revendication 1, dans lequel l'élément de pénétration comprend une canule appropriée pour pénétrer dans un élément souple du récipient de stockage.

3. Dispositif de prélèvement de sang automatique selon la revendication 2, dans lequel la canule est montée de façon mobile par rapport au récipient de stockage.

4. Dispositif de prélèvement de sang automatique selon la revendication 3, dans lequel la canule est disposée sur un chariot, le chariot étant approprié pour effectuer un mouvement de translation par rapport au récipient de stockage, le mouvement de translation du chariot se faisant le long d'un axe du récipient de stockage, dans lequel la canule est alignée avec ledit axe.

5. Dispositif de prélèvement de sang automatique selon la revendication 4, comprenant également un premier et un deuxième élément capteur, dans lequel le premier élément capteur est approprié pour produire un premier signal de position quand le chariot se trouve en position de retrait, où la canule est séparée du récipient de stockage, et dans lequel le deuxième élément capteur est approprié pour produire un deuxième signal de position quand le chariot se trouve en position avancée, où la canule a pénétré dans l'élément souple du récipient de stockage.

6. Dispositif de prélèvement de sang automatique selon l'une quelconque des revendications précédentes, dans lequel le dispositif comprend une pluralité de récipients de stockage disposés dans un tambour monté de manière rotative.

7. Dispositif de prélèvement de sang automatique selon la revendication 6, dans lequel le tambour monté de manière rotative comprend un premier et un deuxième disque essentiellement circulaires, les disques étant parallèles l'un à l'autre et montés sur un axe rotatif raccordé de manière fonctionnelle à un moteur électrique.

8. Dispositif de prélèvement de sang automatique selon la revendication 7, dans lequel chacun des disques essentiellement circulaires comprend plusieurs systèmes appropriés pour soutenir la pluralité de récipients de stockage.

9. Dispositif de prélèvement de sang automatique selon la revendication 8, dans lequel les systèmes destinés à soutenir la pluralité de récipients de stockage comprennent des ouvertures traversantes ménagées dans les disques de manière à ce qu'un récipient de stockage soit maintenu dans le tambour par pénétration dans une ouverture traversante du premier disque et pénétration dans une ouverture traversante correspondante du deuxième disque.

10. Dispositif de prélèvement de sang automatique selon l'une quelconque des revendications 7 à 9, comprenant également un mécanisme d'alignement approprié pour aligner axialement l'élément de pénétration avec un récipient de stockage, le mécanisme d'alignement comprenant un moyen permettant de générer un signal d'alignement quand le récipient de stockage est aligné axialement avec l'élément de pénétration.

11. Dispositif de prélèvement de sang automatique selon la revendication 10, dans lequel le mécanisme d'alignement comprend un émetteur optique et un récepteur optique, l'émetteur et le récepteur optiques étant disposés de manière à pouvoir coupler un faisceau lumineux au travers de parties transparentes d'un disque et détecter les parties transmises dudit faisceau lumineux.

12. Dispositif de prélèvement de sang automatique selon la revendication 11, dans lequel les parties transparentes sont situées dans l'alignement des ouvertures traversantes de l'un des disques.

13. Dispositif de prélèvement de sang automatique selon la revendication 12, dans lequel le signal d'alignement est généré quand le récepteur optique reçoit au moins une partie d'un signal optique transmis, le signal optique étant transmis de l'émetteur optique vers le récepteur optique au travers d'une ouverture traversante d'un troisième disque essentiellement circulaire.

14. Dispositif de prélèvement de sang automatique selon l'une quelconque des revendications 6 à 13, dans lequel le tambour monté de manière rotative est approprié pour recevoir 14 récipients de stockage, un récipient à déchets et un réservoir approprié pour contenir l'anticoagulant, tel que l'héparine.

15. Dispositif de prélèvement de sang automatique selon l'une quelconque des revendications précédentes, dans lequel le moyen de pompage comprend une pompe péristaltique.

16. Dispositif de prélèvement de sang automatique selon l'une quelconque des revendications précédentes, comprenant également une unité de commande centrale destinée à commander le fonctionnement du dispositif de prélèvement de sang.

17. Dispositif de prélèvement de sang automatique selon la revendication 16, comprenant également un moyen de stockage électronique approprié pour stocker des programmes lisibles, lesdits programmes lisibles déterminant, lors de leur exécution par l'unité de commande centrale, le fonctionnement du dispositif de prélèvement de sang.
